# EUROPEAN PATENT APPLICATION

(11) **EP 3 831 384 A1**
(43) Date of publication of application: **09.06.2021**
(21) Application number: 19306550.5
(22) Date of filing: 02.12.2019
(51) Int. Cl.: A61K 31/496, A61K 45/06, A61P 11/06

(54) **USE OF MASITINIB FOR THE TREATMENT OF EOSINOPHILIC ASTHMA**

(71) Applicant: AB Science, 75008 Paris (FR)
(72) Inventor: MOUSSY, Alain, 75006 Paris (FR); KINET, Jean-Pierre, Lexington, MA Massachusetts 02420 (US)
(74) Representative: Icosa

(57) **Abstract**

The present invention relates to a 2-aminoarylthiazole derivative or a pharmaceutically acceptable salt or solvate thereof, in particular masitinib or a pharmaceutically acceptable salt or solvate thereof, for use in the treatment of asthma in a subject in need thereof, wherein the subject has an elevated level of eosinophils. In particular, the present invention relates to a 2-aminoarylthiazole derivative or a pharmaceutically acceptable salt or solvate thereof, in particular masitinib or a pharmaceutically acceptable salt or solvate thereof, for use in the treatment of asthma in a subject with an eosinophil blood count at baseline ranging from 150 cells/µL to 300 cells/µL.

## Description

### FIELD OF INVENTION

The present invention relates to the treatment of asthma in a subject in need thereof, and in particular to the treatment of eosinophilic asthma.

### BACKGROUND OF INVENTION

Asthma is a chronic inflammatory disease of the airways characterized by variable airflow obstruction, airway hyper-responsiveness, and airway inflammation. Asthma being a chronic disease, the aim of asthma treatment is to achieve control of the disease, in particular to lessen symptoms and to reduce asthma exacerbations, thus improving quality of life.

Eosinophils, a type of inflammatory granulocytes, are involved in asthma pathophysiology. Increased numbers of eosinophils have been reported in the bronchoalveolar lavage, in the sputum and/or in the peripheral blood of subjects with asthma. Accumulation of eosinophils, notably in the airways, may lead to inflamed and narrowed airways following degranulation and release of inflammatory mediators such as eosinophil-derived neurotoxin, eosinophil cationic protein, eosinophil peroxidase, and major basic protein. Eosinophilic inflammation is correlated with asthma severity, and notably with frequency of asthma exacerbations. Severe asthma is generally associated with a difficulty to achieve control of the disease.

In many subjects, asthma is a disease well controlled by maintenance medication, including for example corticosteroids, in particular inhaled corticosteroids, and/or bronchodilators. However, in a fraction of subjects suffering from asthma, notably in subjects with elevated eosinophils and in subjects with eosinophilic asthma, asthma remains uncontrolled by such maintenance medication. Thus, in some subjects, asthma is refractory to generally effective maintenance medications such as corticosteroids, in particular inhaled corticosteroids, and/or bronchodilators. Subjects suffering from refractory asthma (also referred to as uncontrolled asthma) are usually administered higher doses of corticosteroids, notably of oral corticosteroids, and may thus become dependent on oral corticoids to achieve asthma control. However, oral corticosteroids can induce severe side effects and their prolonged use should thus be avoided. Moreover, in some subjects, asthma also displays a poor response to oral corticoids.

Therefore, there is still a need for effective treatments of asthma, in particular of asthma difficult to treat with the currently available maintenance medications, for example in subjects with eosinophilic inflammation and/or subjects with eosinophilic asthma. Notably, there is a need for effective treatments achieving control of asthma, for example by reducing asthma exacerbation.

The present invention thus relates to a 2-aminoarylthiazole derivative, in particular masitinib, or a pharmaceutically acceptable salt or solvate thereof, for use in the treatment of asthma in a subject in need thereof, wherein the subject has an elevated level of eosinophils, in particular wherein the subject has an eosinophil blood count at baseline ranging from 150 cells/µL to 300 cells/µL.

### SUMMARY

The present invention relates to a 2-aminoarylthiazole derivative or a pharmaceutically acceptable salt or solvate thereof for use in the treatment of asthma in a subject in need thereof, wherein the subject has an eosinophil blood count at baseline equal to or higher than 150 cells/µL. The present invention also relates to a method for treating asthma in a subject in need thereof, wherein the subject has an eosinophil blood count at baseline equal to or higher than 150 cells/µL, comprising administering to the subject a 2-aminoarylthiazole derivative or a pharmaceutically acceptable salt or solvate.

According to an embodiment, the 2-aminoarylthiazole derivative or a pharmaceutically acceptable salt or solvate thereof is for use in the treatment of asthma in a subject in need thereof, wherein the subject has an eosinophil blood count at baseline ranging from 150 cells/µL to 300 cells/µL. According to an embodiment, the 2-aminoarylthiazole derivative or a pharmaceutically acceptable salt or solvate thereof is for use in the treatment of asthma in a subject in need thereof, wherein the subject has an eosinophil blood count at baseline equal to or higher than 300 cells/µL, preferably higher than 300 cells/µL.

Accordingly, in one embodiment, the present invention relates to a method for treating asthma in a subject in need thereof, wherein the subject has an eosinophil blood count at baseline ranging from 150 cells/µL to 300 cells/µL, comprising administering to the subject a 2-aminoarylthiazole derivative or a pharmaceutically acceptable salt or solvate. In one embodiment, the present invention relates to a method for treating asthma in a subject in need thereof, wherein the subject has an eosinophil blood count at baseline equal to or higher than 300 cells/µL, preferably higher than 300 cells/µL, comprising administering to the subject a 2-aminoarylthiazole derivative or a pharmaceutically acceptable salt or solvate.

According to one embodiment, the asthma is asthma with type 2 inflammation, also known as type 2 asthma.

According to one embodiment, the asthma is uncontrolled. In other words, according to one embodiment, the asthma is refractory to maintenance medication, such as corticosteroids and/or biologics. In one embodiment, the asthma is uncontrolled with inhaled corticosteroids and/or oral corticoids. In other words, in one embodiment, the asthma is refractory to inhaled corticosteroids and/or oral corticoids. In one embodiment, the asthma is uncontrolled with biologics. In other words, in one embodiment, the asthma is refractory to biologics.

In one embodiment, the 2-aminoarylthiazole derivative or a pharmaceutically acceptable salt or solvate thereof for use according to any one of claims 1 to 5, wherein the 2-aminoarylthiazole derivative has the formula (II): 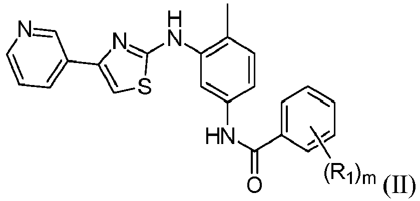 wherein:
- R₁ is selected independently from hydrogen, halogen, (C1 C10) alkyl, (C3 C10) cycloalkyl group, trifluoromethyl, alkoxy, amino, alkylamino, dialkylamino, and a solubilizing group, and
- m is 0-5.

In one embodiment, the 2-aminoarylthiazole derivative or a pharmaceutically acceptable salt or solvate thereof is masitinib or a pharmaceutically acceptable salt or solvate thereof. In one embodiment, the pharmaceutically acceptable salt or solvate of masitinib is masitinib mesilate.

In one embodiment, the 2-aminoarylthiazole derivative or a pharmaceutically acceptable salt or solvate thereof is to be administered orally.

According to one embodiment, the 2-aminoarylthiazole derivative or a pharmaceutically acceptable salt or solvate thereof is to be administered at a dose ranging from about 1 mg/kg/day to about 12 mg/kg/day, preferably from about 3 mg/kg/day to about 6 mg/kg/day. In one embodiment, the 2-aminoarylthiazole derivative or a pharmaceutically acceptable salt or solvate thereof is to be administered at a dose of about 3 mg/kg/day, 4.5 mg/kg/day or 6 mg/kg/day. In one embodiment, the 2-aminoarylthiazole derivative or a pharmaceutically acceptable salt or solvate thereof, is to be administered at an initial dose of about 3 mg/kg/day during at least 4 weeks, then at a dose of about 4.5 mg/kg/day during at least 4 weeks, and at a dose of about 6 mg/kg/day thereafter, with each dose escalation being subjected to toxicity controls.

According to one embodiment, the 2-aminoarylthiazole derivative or a pharmaceutically acceptable salt or solvate thereof is to be administered with at least another pharmaceutically active agent. In one embodiment, the at least another pharmaceutically active agent is selected from the group consisting of biologics, corticosteroids, bronchodilators (including LABAs), leukotriene modifiers, rescue medications and any combinations thereof. In embodiment, the at least another pharmaceutically active agent is a biologic selected from the group consisting of anti-IgE agents and anti-cytokine agents, such as anti-IL-5 agents. In one embodiment, the biologic is an anti-IL-5 agent.

According to one embodiment, the at least another pharmaceutically active agent is administered as a first line treatment and the 2-aminoarylthiazole derivative or a pharmaceutically acceptable salt or solvate thereof is administered as a second line treatment.

### DEFINITIONS

In the present invention, the following terms have the following meanings:
- **"About"** preceding a figure encompasses plus or minus 10%, or less, of the value of said figure. It is to be understood that the value to which the term "about" refers is itself also specifically, and preferably, disclosed.
- **"Asthma exacerbation"** or **"asthma attack"** or **"asthma episode"** or **"asthma flare-up"** are interchangeable expressions referring to an episode characterized by at least one of the following:
   ∘ manifestation and/or progressive worsening (also referred to as deterioration) of asthma symptoms;
   ∘ deterioration of lung function;
   ∘ increased use of rescue medication.
   Examples of asthma symptoms include, without being limited to, wheezing, cough, shortness of breath (also known as dyspnea) and chest tightness. Asthma exacerbation may require a hospital visit and/or stay. Asthma exacerbation may be moderate or severe. In one embodiment, moderate asthma exacerbation is defined as a deterioration in asthma symptoms, a deterioration in lung function and/or an increased use of rescue medication, in particular wherein said deterioration in asthma symptoms, deterioration in lung function and/or increased use of rescue medication last(s) for at least 2 days. In one embodiment, moderate asthma exacerbation is defined as a deterioration in asthma symptoms, a deterioration in lung function and/or an increased use of rescue medication, in particular wherein said deterioration in asthma symptoms, deterioration in lung function and/or increased use of rescue medication last(s) for at least 2 days and require(s) a change in asthma treatment (other than an increase in systemic corticosteroids dose or hospitalization). In one embodiment, severe asthma exacerbation is defined as an asthma worsening requiring a hospitalization and/or an increase from the stable dose of control medication, such as corticosteroids, in particular for at least 3 days.
- **"Asthma control"** or **"clinical control of asthma"** refers to the clinical management of asthma with medication which aims in particular at preventing or lessening airway inflammation, at preventing or lessening asthma symptoms and/or asthma associated limitations, and at preventing asthma exacerbation. For example, in a subject in whom asthma is controlled, asthma induces little or no limitations, such as limitations on regular exercise and/or daily activities; little or no loss of sleep quality such as night-time awakening(s), and does not frequently lead to a need for rescue treatment. Such control medications are also referred to maintenance medications. Control medications may for example reduce inflammation in the airways, or help keep the airways open. Examples of control medications include, without being limited to, corticosteroids, in particular inhaled corticosteroids, leukotriene modifiers, long-acting beta agonists (LABAs), combinations of a corticosteroid and a LABA, and bronchodilators such as theophylline.
- **"Baseline"** as used herein refers to the time preceding the start of the treatment with the 2-aminoarylthiazole derivative, or a pharmaceutically acceptable salt or solvate thereof, as described herein. For example, for a given subject, the eosinophil blood count at baseline is the eosinophil blood count prior to the administration to the subject of a 2-aminoarylthiazole derivative, or a pharmaceutically acceptable salt or solvate thereof, as described herein.
- **"BID"** as used herein in reference with inhaled asthma treatment means twice daily.
- **"Eosinophil"** (sometimes also known as acidophil) refers to a type of proinflammatory white blood cell (WBC), more precisely a granulocyte, characterized notably by a multilobed nucleus and cytoplasmic granulation that is readily stained by eosin. Eosinophils play a role in inflammation and in particular in airway inflammation.
- **"Eosinophil count"** refers to a measure of the number of eosinophils. For example, the number of eosinophils may be measured in a tissue, in a bronchoalveolar lavage, in the sputum or in the blood. As indicated hereinafter, the number of eosinophils measured in the blood is known as the eosinophil blood count.
- **"Eosinophil blood count"** (also known as absolute eosinophil count) refers to the number in the blood of white blood cells that are eosinophils. Methods for assessing the eosinophil blood count of a subject are routinely used in clinical laboratories. For example, the measure may be carried out by counting the number of eosinophils per 100 cells and multiplying the resulting percentage by the white blood cell count (*i.e*., the total number of white blood cells). The eosinophil blood count is usually expressed as a number of cells (*i.e*., eosinophils) per µL of blood or per mm³ of blood.
- **"Exacerbation rate"** refers to the number of asthma exacerbations occurring in a subject over a given period of time. For example, the annual or yearly exacerbation rate refers to the number of asthma exacerbations occurring in a subject over a year.
- **"Lung function"** as used herein reflects airflow, with a decreased lung function reflecting airflow limitation. In one embodiment, lung function, and thus airflow limitation, may be assessed through the measurement of forced expiratory volume (FEV), forced expiratory volume in 1 second (FEVi), percentage of predicted FEVi (which as used herein compares how much a subject with asthma can exhale with how much a subject without asthma could exhale), peak expiratory flow (PEF) and/or forced vital capacity (FVC). Methods for assessing lung function, and thus airflow limitation, are well known and include in particular spirometry.
- **"Pharmaceutically acceptable excipient"** or **"pharmaceutically acceptable carrier"** refers to an excipient or carrier that does not produce an adverse, allergic or other untoward reaction when administered to a mammal, preferably a human. It includes any and all solvents, such as, for example, dispersion media, coatings, antibacterial and antifungal agents, isotonic and absorption delaying agents. A pharmaceutically acceptable excipient or carrier refers to a non-toxic solid, semi-solid or liquid filler, diluent, encapsulating material or formulation auxiliary of any type. For human administration, preparations should meet sterility, pyrogenicity, general safety and purity standards as required by the regulatory offices such as the FDA or EMA.
- **"Rescue medication"** as used herein refers to a quick-acting or fast-acting medication aiming at relieving asthma symptoms immediately. In particular, rescue medication as used herein refers to a quick-acting or fast-acting medication aiming at relieving an asthma exacerbation. Examples of rescue medication include, without being limited to, bronchodilators such as short-acting beta agonists and ipratropium, corticoids such as oral or intravenous corticosteroids.
- **"Subject"** refers to a mammal, preferably a human. According to the present invention, a subject is a mammal, preferably a human, suffering from asthma. In one embodiment, the subject may be a "patient", *i.e.,* a warm-blooded animal, preferably a human, who/which is awaiting the receipt of, or is receiving medical care or was/is/will be the object of a medical procedure, or is monitored for the development of asthma. In one embodiment, the subject is as patient, *i.e.,* a warm-blooded animal, preferably a human, who/which is receiving medical care, in particular maintenance medication, and/or is monitored for asthma.
- **"Therapeutically effective amount"** or **"therapeutically effective dose"** refers to the amount or concentration of a 2-aminoarylthiazole derivative, as defined herein, or a pharmaceutically acceptable salt or solvate thereof, that is aimed at, without causing significant negative or adverse side effects to the subject in need of treatment, bringing about at least one of the following:
   ∘ reducing asthma exacerbation, for example reducing moderate asthma exacerbation, reducing severe asthma exacerbation and/or reducing overall asthma exacerbation;
   ∘ preventing or lessening one or more of the symptoms of asthma;
   ∘ preventing or lessening airway inflammation;
   ∘ preventing or lessening a deterioration of lung function;
   ∘ decreasing the need for rescue medication;
   ∘ decreasing the needed dose of inhaled corticosteroids and/or of long-term oral corticosteroids, thereby promoting a steroid-saving effect;
   ∘ preventing the need for an increase in the dose of maintenance medication;
   ∘ decreasing the frequency of hospital visits and/or admissions, in other words, decreasing the frequency of hospitalization;
   ∘ shortening the length of hospital visits and/or stays, in other words, shortening the length of hospitalization; and/or
   ∘ improving quality of life.
   According to one embodiment, the therapeutically effective amount or therapeutically effective dose refers to the amount or concentration of a 2-aminoarylthiazole derivative, as defined herein, or a pharmaceutically acceptable salt or solvate thereof, that is aimed at, without causing significant negative or adverse side effects to the subject in need of treatment, reducing asthma exacerbation, in particular the rate of asthma exacerbation, such as the yearly rate of asthma exacerbation. In one embodiment, asthma exacerbation is moderate asthma exacerbation and/or severe asthma exacerbation, and the rate of asthma exacerbation is the rate of moderate asthma exacerbation and/or the rate of severe asthma exacerbation (respectively), such as the yearly rate of moderate asthma exacerbation and/or the yearly rate of severe asthma exacerbation. In one embodiment, asthma exacerbation is overall asthma exacerbation, including both moderate and severe asthma exacerbation, and the rate of asthma exacerbation is the rate of overall asthma exacerbation, such as the yearly rate of overall asthma exacerbation.
- **"Treating"** or **"Treatment"** refers to a therapeutic treatment, to a prophylactic (or preventative) treatment, or to both a therapeutic treatment and a prophylactic (or preventative) treatment, wherein the object is to prevent, reduce or slow down (lessen) one or more of the symptoms or manifestations of asthma in a subject in need thereof, preferably the object is to reduce asthma exacerbation, such as moderate asthma exacerbation, severe asthma exacerbation and/or overall asthma exacerbation. In one embodiment, the object of the treatment according to the present application is to bring about at least one of the following:
   ∘ reducing asthma exacerbation, for example reducing moderate asthma exacerbation, reducing severe asthma exacerbation and/or reducing overall asthma exacerbation;
   ∘ preventing or lessening one or more of the symptoms of asthma;
   ∘ preventing or lessening airway inflammation;
   ∘ preventing or lessening a deterioration of lung function;
   ∘ decreasing the need for rescue medication;
   ∘ decreasing the needed dose of inhaled corticosteroids and/or of long-term oral corticosteroids, thereby promoting a steroid-saving effect;
   ∘ preventing the need for an increase in the dose of maintenance medication;
   ∘ decreasing the frequency of hospital visits and/or admissions, in other words, decreasing the frequency of hospitalization;
   ∘ shortening the length of hospital visits and/or stays, in other words, shortening the length of hospitalization; and/or
   ∘ improving quality of life.
   Accordingly, a subject suffering from asthma is considered as successfully "treated", if, after receiving a therapeutically effective amount of a 2-aminoarylthiazole derivative as defined herein, or a pharmaceutically acceptable salt or solvate thereof, the subject benefits from at least one of the following:
   ∘ a reduction of asthma exacerbation, in particular of the rate of asthma exacerbation, such as the rate of moderate asthma exacerbation, severe asthma exacerbation and/or overall asthma exacerbation;
   ∘ a lessening of one or more of the symptoms of asthma, such as wheezing, cough, shortness of breath (also known as dyspnea) and chest tightness;
   ∘ a lessening of airway inflammation;
   ∘ a decrease of the need for rescue medication;
   ∘ a decrease of the frequency of hospital visits and/or admissions, in other words, a decrease of the frequency of hospitalization;
   ∘ a shortening of the length of hospital visits and/or stays, in other words, a shortening of the length of hospitalization; and/or
   ∘ an improvement of the quality of life.
- **"Type 2 asthma"** or **"asthma with type 2 inflammation"** refers to a phenotype of asthma characterized by the production of cytokines such as interleukin (IL)-4, IL-5 and IL-13, which are often produced by the adaptive immune system on recognition of allergens. Type 2 inflammation is often characterized by eosinophils, and may be accompanied by atopy, whereas non-type 2 inflammation is often characterized by neutrophils. In subjects with mild or moderate asthma, type 2 inflammation rapidly improves when inhaled corticosteroid (ICS) are taken regularly and correctly. In severe asthma, type 2 inflammation is relatively refractory to high dose of ICS. Severe type 2 asthma may respond to oral corticosteroid (OCS) but their serious adverse effects mean that alternative treatments should be sought. The possibility of refractory type 2 inflammation should be considered if blood eosinophils ≥150/µL, is found while the subject is taking high-dose ICS or daily OCS [Global Initiative for Asthma. Global Strategy for Asthma Management and Prevention, 2019. Available from: www. ginasthma.org].
- **"Uncontrolled asthma"** also known as **"refractory asthma"** refers to asthma that is not controlled by maintenance medications, in particular bronchodilators and/or corticoids, such as inhaled corticoids or even in some cases oral corticoids. In other words, in a subject suffering from uncontrolled asthma or refractory asthma, the maintenance medications do not achieve their aim, for example of preventing or lessening airway inflammation, preventing or lessening asthma symptoms and/or asthma associated limitations, and preventing asthma exacerbation. In one embodiment, uncontrolled asthma is defined by the presence of at least one of the following: limitations of activities, loss of sleep quality and in particular night-time awakening(s), and/or need for rescue treatment at least twice per week.

### DETAILED DESCRIPTION

The present invention relates to a 2-aminoarylthiazole derivative as described herein, or a pharmaceutically acceptable salt or solvate thereof, for use in the treatment of asthma in a subject in need thereof, wherein the subject has an elevated level of eosinophils, in particular an elevated level of eosinophils at baseline.

According to one embodiment, the level of eosinophils is the level or number or percentage of eosinophils in a biological sample from the subject.

Examples of biological samples include, without being limited to, tissue sample, blood sample, sputum sample, bronchoalveolar lavage fluid.

In one embodiment, the level of eosinophils is the level or number or percentage of eosinophils in a sputum sample, in a tissue sample such as a lung tissue sample or a bronchus tissue sample, in a bronchoalveolar lavage fluid sample or in a blood sample.

According to the present invention, eosinophilic asthma (EA), sometimes called severe eosinophilic asthma (SEA), refers to a subtype of asthma characterized by elevated levels of eosinophils. Thus, one object of the invention is a 2-aminoarylthiazole derivative as described herein, or a pharmaceutically acceptable salt or solvate thereof, for use in the treatment of eosinophilic asthma (EA) in a subject in need thereof. Another object of the invention is a 2-aminoarylthiazole derivative as described herein, or a pharmaceutically acceptable salt or solvate thereof, for use in the treatment of severe eosinophilic asthma (SEA) in a subject in need thereof.

According to one embodiment, the level of eosinophils is the eosinophil blood count. In one embodiment, according to the present application, an elevated level of eosinophils is an eosinophil blood count equal to or higher than 150 cells/µL (that is to say 150 eosinophils per µL of blood). In one embodiment, an elevated level of eosinophils is an eosinophil blood count ranging from 150 cells/µL to 300 cells/µL. In one embodiment, an elevated level of eosinophils is an eosinophil blood count equal to or higher than 300 cells/µL (that is to say 300 eosinophils per µL of blood), preferably higher than 300 cells/µL.

One object of the invention is a 2-aminoarylthiazole derivative as described herein, or a pharmaceutically acceptable salt or solvate thereof, for use in the treatment of asthma in a subject in need thereof, wherein the subject has an eosinophil blood count at baseline equal to or higher than 150 cells/µL. In one embodiment, the 2-aminoarylthiazole derivative as described herein, or a pharmaceutically acceptable salt or solvate thereof, is for use in the treatment of eosinophilic asthma in a subject in need thereof, wherein the subject has an eosinophil blood count at baseline equal to or higher than 150 cells/µL.

One object of the invention is a 2-aminoarylthiazole derivative as described herein, or a pharmaceutically acceptable salt or solvate thereof, for use in the treatment of asthma in a subject in need thereof, wherein the subject has an eosinophil blood count at baseline ranging from 150 cells/µL to 300 cells/µL. In one embodiment, the 2-aminoarylthiazole derivative as described herein, or a pharmaceutically acceptable salt or solvate thereof, is for use in the treatment of eosinophilic asthma in a subject in need thereof, wherein the subject has an eosinophil blood count at baseline ranging from 150 cells/µL to 300 cells/µL.

One object of the invention is a 2-aminoarylthiazole derivative as described herein, or a pharmaceutically acceptable salt or solvate thereof, for use in the treatment of asthma in a subject in need thereof, wherein the subject has an eosinophil blood count at baseline equal to or higher than 300 cells/µL, preferably higher than 300 cells/µL. In one embodiment, the 2-aminoarylthiazole derivative as described herein, or a pharmaceutically acceptable salt or solvate thereof, is for use in the treatment of eosinophilic asthma in a subject in need thereof, wherein the subject has an eosinophil blood count at baseline equal to or higher than 300 cells/µL, preferably higher than 300 cells/µL.

According to one embodiment, the subject to be treated as described herein is suffering from severe asthma, in particular severe eosinophilic asthma. Asthma severity is conventionally classified by the presence of clinical features before treatment is started, the airflow limitation and/or by the amount of daily medication required for optimal treatment [Global Strategy for Asthma Management and Prevention, Global Initiative for Asthma (GINA) 2007; http://www.ginasthma.org].

Asthma severity may for example be assessed by taking into consideration the frequency and intensity of asthma symptoms such as wheezing, cough, shortness of breath (also known as dyspnea) and chest tightness; the level of airflow limitation which may be assessed for example through the measurement of forced expiratory volume in 1 second (FEV₁) and/or the measurement of peak expiratory flow (PEF); the frequency of asthma exacerbations or the rate of asthma exacerbation; the quality of sleep and/or the frequency of nocturnal asthma symptoms such as night time awakening; the limitation of physical activities.

Asthma severity may also be assessed according to the intensity of maintenance treatment required to achieve good asthma control.

In one embodiment, severe asthma is defined as asthma requiring high intensity treatment by maintenance medications, such as for example oral corticosteroids, to maintain good control, or as asthma not achieving good control despite high intensity treatment, such as for example oral corticosteroids.

According to one embodiment, severe asthma as described herein is severe persistent asthma.

In one embodiment, severe persistent asthma refers to asthma which remains difficult to control despite optimal management, treatment compliance by the subject, and extensive re-evaluation of diagnosis following an observation period of at least 6 months by an asthma specialist.

In one embodiment, severe persistent asthma is characterized by the presence of daily asthma symptoms (such as wheezing, cough, shortness of breath (also known as dyspnea) and chest tightness), frequent asthma exacerbations (such as at least 2 asthma exacerbations per year), frequent nocturnal asthma symptoms such as nighttime awakening(s), limitation of physical activities, FEVi or PEF ≤ 60% predicted FEVi or PEF, and/or PEF or FEVi variability > 30%.

According to one embodiment, the subject to be treated as described herein is suffering from asthma, in particular severe and/or eosinophilic asthma, that is not under control.

Thus, in one embodiment, the subject to be treated as described herein is suffering from uncontrolled asthma, in particular uncontrolled severe and/or eosinophilic asthma. In other words, the subject to be treated as described herein is suffering from asthma, in particular severe and/or eosinophilic asthma, refractory to maintenance medication.

Therefore, according to one embodiment, the treatment of asthma with a 2-aminoarylthiazole derivative as described herein, or a pharmaceutically acceptable salt or solvate thereof, is a second line (or further line) of treatment.

In one embodiment, the maintenance medication is a corticosteroid. Thus, in one embodiment, the subject to be treated as described herein is suffering from asthma, in particular severe and/or eosinophilic asthma, uncontrolled with corticosteroids. In other words, in one embodiment, the subject to be treated as described herein is suffering from asthma, in particular severe and/or eosinophilic asthma, refractory to corticosteroids.

Examples of corticoids used as maintenance medication for the treatment of asthma include, without being limited to, fluticasone, budesonide, mometasone, beclomethasone, prednisone, prednisolone, betamethasone and dexamethasone.

In one embodiment, at any time, if two or more corticosteroids are administered concomitantly, then the corticosteroid dose considered during the overlap period will be sum of all individual prednisone equivalent dose.

In one embodiment, the maintenance medication is an inhaled corticosteroid (ICS). Thus, in one embodiment, the subject to be treated as described herein is suffering from asthma, in particular severe and/or eosinophilic asthma, uncontrolled with inhaled corticosteroids. In other words, in one embodiment, the subject to be treated as described herein is suffering from asthma, in particular severe and/or eosinophilic asthma, refractory to inhaled corticosteroids.

Examples of inhaled corticoids used as maintenance medication for the treatment of asthma include, without being limited to, fluticasone, budesonide, mometasone and beclomethasone.

In one embodiment, the maintenance medication is selected from the group comprising or consisting of fluticasone, budesonide, mometasone, beclomethasone and any combinations thereof.

In one embodiment, as used in the present application, inhaled corticoids also include combination of a corticoid and another agent, in particular a long-acting beta agonist (LABA).

Examples of inhaled corticoids used as maintenance medication for the treatment of asthma comprising or consisting of a corticoid in combination with a LABA include, without being limited to, a combination of fluticasone and salmeterol, a combination of budesonide and formoterol and a combination of mometasone and formoterol.

In one embodiment, the maintenance medication is selected from the group comprising or consisting of a combination of fluticasone and salmeterol (for example DPI (dry-powder inhaler) formulation: 500/50 µg BID (BID meaning twice daily) or MDI (metered dose inhaler) formulation: 460/42 µg breath-activated digital inhaler (BID), a combination of budesonide and formoterol (for example 320/9 µg BID) and a combination of mometasone and formoterol (for example (400/10 µg BID). In one embodiment, the maintenance medication is a combination of fluticasone and salmeterol (for example DPI formulation: 500/50 µg BID or MDI formulation: 460/42 µg BID), a combination of budesonide and formoterol (for example 320/9 µg BID), or a combination of mometasone and formoterol (for example (400/10 µg BID), preferably administered for at least 6, 9, 12, 15, 18 or 24 months, more preferably administered for at least 12 months.

In one embodiment, the maintenance medication is an oral corticosteroid (OCS). Thus, in one embodiment, the subject to be treated as described herein is suffering from asthma, in particular severe and/or eosinophilic asthma, uncontrolled with oral corticosteroids. In other words, in one embodiment, the subject to be treated as described herein is suffering from asthma, in particular severe and/or eosinophilic asthma, refractory to oral corticosteroids.

Examples of oral corticoids used as maintenance medication for the treatment of asthma include, without being limited to, prednisone, prednisolone, betamethasone and dexamethasone.

In one embodiment, at any time, if two or more corticosteroids are administered concomitantly, then the corticosteroid dose considered during the overlap period will be sum of all individual prednisone equivalent dose.

In one embodiment, the maintenance medication is selected from the group comprising or consisting of prednisone, prednisolone, betamethasone, dexamethasone and any combinations thereof.

In one embodiment, the maintenance medication is prednisone. In one embodiment, the maintenance medication is prednisone administered at a daily dose of at least 2.5 mg, 5 mg, 7.5 mg, 10 mg, 12.5 mg, 15 mg, 17.5 mg, 20 mg, preferably for at least 1, 2, 3, 4, 5 or 6 months. In one embodiment, the maintenance medication is prednisone administered at a daily dose of at least 5 mg, preferably for at least 3 months. In one embodiment, the maintenance medication is prednisone administered at a daily dose of at least 7.5 mg, preferably for at least 3 months. In one embodiment, the maintenance medication is prednisone administered at a daily dose of at least 15 mg, preferably for at least 3 months.

According to one embodiment, the administration of a 2-aminoarylthiazole derivative as described herein, or a pharmaceutically acceptable salt or solvate thereof, to the subject to be treated as described herein permits a reduction in the subject's intake of inhaled corticosteroids and/or of long-term oral corticosteroids as described hereinabove; that is to say, the administration of a 2-aminoarylthiazole derivative as described herein, or a pharmaceutically acceptable salt or solvate thereof, promotes a steroid-saving effect.

Other examples of maintenance medication may include, without being limited to, biologics, bronchodilators and leukotriene modifiers. Non-limiting examples of biologics, bronchodilators and leukotriene modifiers are listed herein.

In one embodiment, the maintenance medication is a biologic. In one embodiment, the maintenance medication is a biologic selected from the group comprising or consisting of dupilumab, mepolizumab, reslizumab, benralizumab and any mixes thereof.

In one embodiment, the subject in need of treatment is a male. In another embodiment, the subject in need of treatment is a female.

According to one embodiment, the subject in need of treatment is an adult. According to the present invention, an adult is a subject above the age of 18, 19, 20 or 21 years. In one embodiment, the subject in need of treatment is older than 20, 25, or 30 years.

According to one embodiment, the subject in need of treatment is a child. According to the present invention, a child is a subject below 21, 20, 19 or 18 years.

In one embodiment, the subject is as patient, *i.e.,* a human who is receiving medical care, in particular maintenance medication, and/or is monitored for asthma. In one embodiment, the subject is as patient, *i.e.,* a human who is receiving medical care, in particular maintenance medication, and is monitored for asthma.

In one embodiment, the subject is suffering from severe asthma as described hereinabove.

In one embodiment, prior to treatment with the 2-aminoarylthiazole derivative of the invention (*i.e.,* at baseline), the subject is receiving corticoids, either inhaled corticoids, inhaled corticoids in combination with a long-acting beta agonist (LABA) or oral corticoids. In one embodiment, prior to treatment with the 2-aminoarylthiazole derivative of the invention, the subject is receiving oral corticoids, for example prednisone, at a minimal daily dose of at least 5 mg, 7.5 mg, 10 mg, 12.5 mg, 15 mg, 17.5 mg, 20 mg, preferably at least 7.5 mg. In one embodiment, prior to treatment with the 2-aminoarylthiazole derivative of the invention, the subject is receiving inhaled corticoids in combination with a long-acting beta agonist (LABA), such as, for example a combination of fluticasone and salmeterol (for example DPI (dry-powder inhaler) formulation: 500/50 µg BID (BID meaning twice daily) or MDI (metered dose inhaler) formulation: 460/42 µg breath-activated digital inhaler (BID), a combination of budesonide and formoterol (for example 320/9 µg BID), or a combination of mometasone and formoterol (for example (400/10 µg BID).

In one embodiment, prior to treatment with the 2-aminoarylthiazole derivative of the invention, the subject has been receiving corticoids, either inhaled corticoids, inhaled corticoids in combination with a long-acting beta agonist (LABA) or oral corticoids, for at least 1, 2, 3, 4, 5, or 6 months, preferably at least 3 months. In one embodiment, prior to treatment with the 2-aminoarylthiazole derivative of the invention, the subject has been receiving oral corticoids, for example prednisone, at a minimal daily dose of at least 5 mg, 7.5 mg, 10 mg, 12.5 mg, 15 mg, 17.5 mg, 20 mg, preferably at least 7.5 mg, for at least 1, 2, 3, 4, 5, or 6 months, preferably at least 3 months. In one embodiment, prior to treatment with the 2-aminoarylthiazole derivative of the invention, the subject has been receiving inhaled corticoids in combination with a long-acting beta agonist (LABA), such as, for example a combination of fluticasone and salmeterol (for example DPI (dry-powder inhaler) formulation: 500/50 µg BID (BID meaning twice daily) or MDI (metered dose inhaler) formulation: 460/42 µg breath-activated digital inhaler (BID), a combination of budesonide and formoterol (for example 320/9 µg BID), or a combination of mometasone and formoterol (for example (400/10 µg BID), for at least 3, 6, 9, 12, 15, 18 or 24 months, preferably for at least 12 months.

In one embodiment, in the year preceding treatment with the 2-aminoarylthiazole derivative of the invention, the subject received corticoids, either inhaled corticoids, inhaled corticoids in combination with a long-acting beta agonist (LABA) or oral corticoids, for at least one period of at least 7, 14, 21, 28, 35 days, preferably at least 21 days. In one embodiment, in the year preceding treatment with the 2-aminoarylthiazole derivative of the invention, the subject received oral corticoids, for example prednisone, at a minimal daily dose of at least 5 mg, 7.5 mg, 10 mg, 12.5 mg, 15 mg, 17.5 mg, 20 mg, preferably at least 7.5 mg, for at least one period of at least 7, 14, 21, 28, 35 days, preferably at least 21 days. for at least one period of at least 7, 14, 21, 28, 35 days, preferably at least 21 days.

In one embodiment, prior to treatment with the 2-aminoarylthiazole derivative of the invention (*i.e.,* at baseline), the subject is receiving a maintenance medication, such as, for example, biologics, bronchodilators and leukotriene modifiers. Non-limiting examples of biologics, bronchodilators and leukotriene modifiers are listed herein.

In one embodiment, prior to treatment with the 2-aminoarylthiazole derivative of the invention (*i.e.,* at baseline), the subject has been receiving a maintenance medication, such as, for example, biologics, bronchodilators and leukotriene modifiers. Non-limiting examples of biologics, bronchodilators and leukotriene modifiers are listed herein.

In one embodiment, in the year preceding treatment with the 2-aminoarylthiazole derivative of the invention, the subject received a maintenance medication, such as, for example, biologics, bronchodilators and leukotriene modifiers. Non-limiting examples of biologics, bronchodilators and leukotriene modifiers are listed herein.

In one embodiment, prior to treatment with the 2-aminoarylthiazole derivative of the invention (*i.e*., at baseline), the subject has a FEVi ranging from 35% (included) to 80% (excluded) of the predicted normal value. Thus, in one embodiment, prior to treatment with the 2-aminoarylthiazole derivative of the invention (*i.e*., at baseline), the subject has a FEV₁ ≥ 35% to < 80%.

In one embodiment, the subject has had at least two asthma exacerbations in the 3, 6, 9, 12, 15, 18 or 24 months, preferably 12 months preceding treatment with the 2-aminoarylthiazole derivative of the invention. In one embodiment, the subject has had at least two asthma exacerbations, including at least one severe asthma exacerbation, in the 3, 6, 9, 12, 15, 18 or 24 months, preferably 12 months preceding treatment with the 2-aminoarylthiazole derivative of the invention. In one embodiment, the subject has had at least two severe asthma exacerbations in the 3, 6, 9, 12, 15, 18 or 24 months, preferably 12 months preceding treatment with the 2-aminoarylthiazole derivative of the invention.

In one embodiment, the subject to be treated according to the present invention, in particular the subject with an elevated level of eosinophils as defined hereinabove, is a subject with type 2 asthma, that is to say a subject with asthma presenting with type 2 inflammation.

In the present invention, a 2-aminoarylthiazole derivative refers to a compound characterized by the presence of a thiazolyl group substituted on position 2 (*i.e*., between the heterocyclic nitrogen and sulfur atoms) by a secondary or tertiary amine, wherein the nitrogen atom of the amine is substituted by at least one aryl group.

According to one embodiment, the aryl group is substituted by an arylamide group (*i.e*., -NH-CO-aryl).

In one embodiment, the 2-aminoarylthiazole derivative of the invention has the following formula (I): 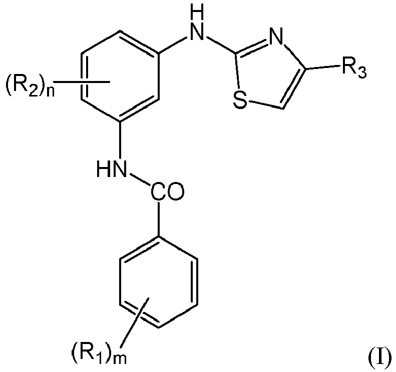 wherein:
- **R₁** and **R₂** are selected independently from hydrogen, halogen, (C₁-C₁₀) alkyl, (C₃-C₁₀) cycloalkyl group, trifluoromethyl, alkoxy, cyano, dialkylamino and a solubilizing group;
- **m** is 0-5;
- **n** is 0-4;
- **R₃** is one of the following:
   (i) an aryl group (such as phenyl), the aryl group being optionally substituted by one or more substituents such as halogen, (C₁-C₁₀) alkyl group, trifluoromethyl, cyano and alkoxy;
   (ii) a heteroaryl group (such as 2, 3, or 4-pyridyl group), the heteroaryl group being optionally substituted by one or more substituents such as halogen, (C₁-C₁₀) alkyl group, trifluoromethyl and alkoxy;
   (iii) a five-membered ring aromatic heterocyclic group (such as, for example, 2-thienyl, 3-thienyl, 2-thiazolyl, 4-thiazolyl, 5-thiazolyl), the aromatic heterocyclic group being optionally substituted by one or more substituents such as halogen, (C₁-C₁₀) alkyl group, trifluoromethyl, and alkoxy.

Thus, in one embodiment, the 2-aminoarylthiazole derivative of the invention or a pharmaceutically acceptable salt or solvate thereof is a 2-aminoarylthiazole derivative of formula (I) or a pharmaceutically acceptable salt or solvate thereof.

In one embodiment, the 2-aminoarylthiazole derivative of the invention has the following formula (II): 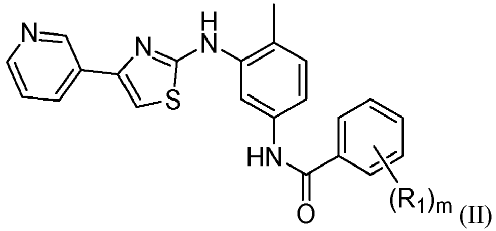 wherein:
- **R₁** is selected independently from hydrogen, halogen, (C₁-C₁₀) alkyl, (C₃-C₁₀) cycloalkyl group, trifluoromethyl, alkoxy, amino, alkylamino, dialkylamino, and a solubilizing group, and
- **m** is 0-5.

Thus, in one embodiment, the 2-aminoarylthiazole derivative of the invention or a pharmaceutically acceptable salt or solvate thereof is a 2-aminoarylthiazole derivative of formula (II) or a pharmaceutically acceptable salt or solvate thereof.

As used herein, the term **"aryl group"** refers to a polyunsaturated, aromatic hydrocarbyl group having a single ring (*i.e.,* phenyl) or multiple aromatic rings fused together (*e.g.,* naphtyl) or linked covalently, typically containing 5 to 12 atoms; preferably 6 to 10, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Examples of suitable aryl groups include, without being limited to, phenyl, tolyl, anthracenyl, fluorenyl, indenyl, azulenyl, and naphthyl, as well as benzo-fused carbocyclic moieties such as 5,6,7,8-tetrahydronaphthyl. An aryl group can be unsubstituted or substituted with one or more substituents. In one embodiment, the aryl group is a monocyclic ring, wherein the ring comprises 6 carbon atoms, referred to herein as "(C₆) aryl".

As used herein, the term **"alkyl group"** refers to a saturated straight chain or branched non-cyclic hydrocarbon having from 1 to 10 carbon atoms, preferably from 1 to 6 carbon atoms. Representative saturated straight chain alkyls include, without being limited to, methyl, ethyl, n-propyl, n-butyl, n-pentyl, n-hexyl, n-heptyl, n-octyl, n-nonyl and n-decyl. Saturated branched alkyls include, without being limited to, isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl. Alkyl groups included in compounds of the present invention may be optionally substituted with one or more substituents.

As used herein, the term **"alkoxy"** refers to an alkyl group which is attached to another moiety by an oxygen atom. Examples of alkoxy groups include, without being limited to, methoxy, isopropoxy, ethoxy, tert-butoxy. Alkoxy groups may be optionally substituted with one or more substituents.

As used herein, the term **"cycloalkyl"** refers to a saturated cyclic alkyl radical having from 3 to 10 carbon atoms. Representative cycloalkyls include cyclopropyl, 1-methylcyclopropyl, cyclobutyl, cyclopentyl, cyclohexyl, cycloheptyl, cyclooctyl, cyclononyl, and cyclodecyl. Cycloalkyl groups can be optionally substituted with one or more substituents.

As used herein, the term **"halogen"** refers to -F, -Cl, -Br or -I.

As used herein, the term **"heteroaryl"** refers to a monocyclic or polycyclic heteroaromatic ring comprising carbon atom ring members and one or more heteroatom ring members (such as, for example, oxygen, sulfur or nitrogen). Typically, a heteroaryl group has from 1 to about 5 heteroatom ring members and from 1 to about 14 carbon atom ring members. Representative heteroaryl groups include, without being limited to, pyridyl, 1-oxo-pyridyl, furanyl, benzo[1,3]dioxolyl, benzo[1,4]dioxinyl, thienyl, pyrrolyl, oxazolyl, imidazolyl, thiazolyl, isoxazolyl, quinolinyl, pyrazolyl, isothiazolyl, pyridazinyl, pyrimidinyl, pyrazinyl, triazinyl, triazolyl, thiadiazolyl, isoquinolinyl, indazolyl, benzoxazolyl, benzofuryl, indolizinyl, imidazopyridyl, tetrazolyl, benzimidazolyl, benzothiazolyl, benzothiadiazolyl, benzoxadiazolyl, indolyl, tetrahydroindolyl, azaindolyl, imidazopyridyl, quinazolinyl, purinyl, pyrrolo[2,3]pyrimidinyl, pyrazolo[3,4]pyrimidinyl, imidazo[1,2-a]pyridyl, and benzo(b)thienyl. A heteroatom may be substituted with a protecting group known to those of ordinary skill in the art, for example, the hydrogen on a nitrogen may be substituted with a tert-butoxycarbonyl group. Heteroaryl groups may be optionally substituted with one or more substituents. In addition, nitrogen or sulfur heteroatom ring members may be oxidized. In one embodiment, the heteroaromatic ring is selected from 5-8 membered monocyclic heteroaryl rings. The point of attachment of a heteroaromatic or heteroaryl ring to another group may be at either a carbon atom or a heteroatom of the heteroaromatic or heteroaryl rings.

As used herein, the term **"heterocycle"** refers collectively to heterocycloalkyl groups and heteroaryl groups.

As used herein, the term **"heterocycloalkyl"** refers to a monocyclic or polycyclic group having at least one heteroatom selected from O, N or S, and which has 2-11 carbon atoms, which may be saturated or unsaturated, but is not aromatic. Examples of heterocycloalkyl groups include, without being limited to, piperidinyl, piperazinyl, 2-oxopiperazinyl, 2-oxopiperidinyl, 2-oxopyrrolidinyl, 4-piperidonyl, pyrrolidinyl, hydantoinyl, valerolactamyl, oxiranyl, oxetanyl, tetrahydropyranyl, tetrahydrothiopyranyl, tetrahydropyrindinyl, tetrahydropyrimidinyl, tetrahydrothiopyranyl sulfone, tetrahydrothiopyranyl sulfoxide, morpholinyl, thiomorpholinyl, thiomorpholinyl sulfoxide, thiomorpholinyl sulfone, 1,3-dioxolane, tetrahydrofuranyl, dihydrofuranyl-2-one, tetrahydrothienyl, and tetrahydro-1,1-dioxothienyl. Typically, monocyclic heterocycloalkyl groups have 3 to 7 members. Preferred 3 to 7 membered monocyclic heterocycloalkyl groups are those having 5 or 6 ring atoms. A heteroatom may be substituted with a protecting group known to those of ordinary skill in the art, for example, the hydrogen on a nitrogen may be substituted with a tert-butoxycarbonyl group. Furthermore, heterocycloalkyl groups may be optionally substituted with one or more substituents. In addition, the point of attachment of a heterocyclic ring to another group may be at either a carbon atom or a heteroatom of a heterocyclic ring. Only stable isomers of such substituted heterocyclic groups are contemplated in this definition.

As used herein, the term **"substituent"** or **"substituted"** means that a hydrogen radical on a compound or group is replaced with any desired group that is substantially stable to reaction conditions in an unprotected form or when protected using a protecting group. Examples of preferred substituents include, without being limited to, halogen (chloro, iodo, bromo, or fluoro); alkyl; alkenyl; alkynyl; hydroxy; alkoxy; nitro; thiol; thioether; imine; cyano; amido; phosphonato; phosphine; carboxyl; thiocarbonyl; sulfonyl; sulfonamide; ketone; aldehyde; ester; oxygen (-O); haloalkyl (*e.g.,* trifluoromethyl); cycloalkyl, which may be monocyclic or fused or non-fused polycyclic (*e.g.,* cyclopropyl, cyclobutyl, cyclopentyl, or cyclohexyl), or a heterocycloalkyl, which may be monocyclic or fused or non-fused polycyclic (*e.g.,* pyrrolidinyl, piperidinyl, piperazinyl, morpholinyl, or thiazinyl), monocyclic or fused or non-fused polycyclic aryl or heteroaryl (*e.g.,* phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); CO₂CH₃; CONH2; OCH2CONH2; NH2; SO₂NH₂; OCHF₂; CF₃; OCF₃; and such moieties may also be optionally substituted by a fused-ring structure or bridge, for example -OCH₂O-. These substituents may optionally be further substituted with a substituent selected from such groups. In certain embodiments, the term "substituent" or the adjective "substituted" refers to a substituent selected from the group consisting of an alkyl, an alkenyl, an alkynyl, an cycloalkyl, an cycloalkenyl, a heterocycloalkyl, an aryl, a heteroaryl, an arylalkyl, a heteroarylalkyl, a haloalkyl,-C(O)NR₁₁R₁₂, -NR₁₃C(O)R₁₄, a halo, -OR₁₃, cyano, nitro, a haloalkoxy, -C(O)R₁₃,-NR₁₁R₁₂, -SR₁₃, -C(O)OR₁₃, -OC(O)R₁₃, -NR₁₃C(O)NR₁₁R₁₂, -OC(O)NR₁₁R₁₂,-NR₁₃C(O)OR₁₄, -S(O)rR₁₃, -NR₁₃S(O)rR₁₄, -OS(O)rR₁₄, S(O)rNR₁₁R₁₂, -O, -S, and -N-R₁₃, wherein r is 1 or 2; R₁₁ and R₁₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl; or R₁₁ and R₁₂ taken together with the nitrogen to which they are attached is optionally substituted heterocycloalkyl or optionally substituted heteroaryl; and R₁₃ and R₁₄ for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl. In certain embodiments, the term "substituent" or the adjective "substituted" refers to a solubilizing group.

As used herein, the term **"solubilizing group"** refers to any group which can be substantially ionized and that enables the compound to be soluble in a desired solvent, such as, for example, water or water-containing solvent ("water-solubilizing group"). Furthermore, the solubilizing group can be one that increases the compound or complex's lipophilicity. In one embodiment, the solubilizing group is selected from alkyl group substituted with one or more heteroatoms such as N, O, S, each optionally substituted with alkyl group substituted independently with alkoxy, amino, alkylamino, dialkylamino, carboxyl, cyano, or substituted with cycloheteroalkyl or heteroaryl, or a phosphate, or a sulfate, or a carboxylic acid. In one embodiment, by "solubilizing group" it is referred herein to one of the following:
- an alkyl, cycloalkyl, aryl, heteroaryl group comprising either at least one nitrogen or oxygen heteroatom or which group is substituted by at least one amino group or oxo group;
- an amino group which may be a saturated cyclic amino group which may be substituted by a group consisting of alkyl, alkoxycarbonyl, halogen, haloalkyl, hydroxyalkyl, amino, monoalkylamino, dialkylamino, carbamoyl, monoalkylcarbamoyl and dialkylcarbamoyl;
- one of the structures a) to i) shown below, wherein the wavy line and the arrow line correspond to the point of attachment to the core structure of the 2-aminoarylthiazole derivative of the invention, for example of formula (I) or (II):

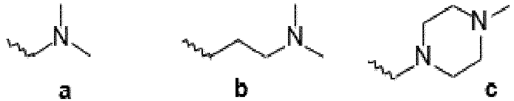

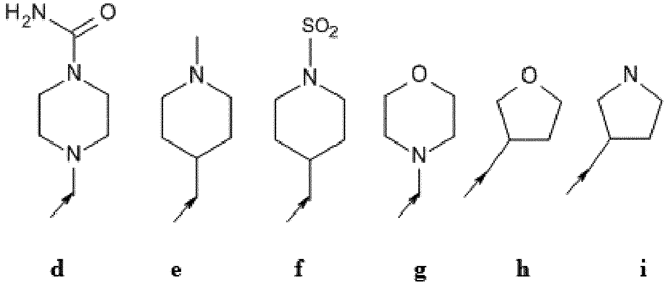

As used herein, **"pharmaceutically acceptable** salt" refers to a salt of a free acid or a free base which is not biologically undesirable and is generally prepared by reacting the free base with a suitable organic or inorganic acid or by reacting the free acid with a suitable organic or inorganic base. Suitable acid addition salts are formed from acids that form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2 napsylate, nicotinate, nitrate, orotate, oxalate, palmitate, pamoate, phosphate/hydrogen, phosphate/dihydrogen, phosphate, pyroglutamate, saccharate, stearate, succinate, tannate, tartrate, tosylate, trifluoroacetate and xinofoate salts. Suitable base salts are formed from bases that form non-toxic salts. Examples include the aluminium, arginine, benzathine, calcium, choline, diethylamine, diolamine, glycine, lysine, magnesium, meglumine, olamine, potassium, sodium, tromethamine, 2 (diethylamino)ethanol, ethanolamine, morpholine, 4 (2 hydroxyethyl)morpholine and zinc salts. Hemi salts of acids and bases may also be formed, e.g., hemi sulphate and hemi calcium salts.

In one embodiment, pharmaceutically acceptable salts are pharmaceutically acceptable acid addition salts, for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic, in particular methanesulfonic acid, or aromatic sulfonic acids, for example benzene-, p-toluene- or naphthalene-2-sulfonic acid.

In one embodiment, the pharmaceutically acceptable salt of the 2-aminoarylthiazole derivative of the invention is mesilate.

Unless otherwise indicated, the term **"mesilate"** is used herein to refer to a salt of methanesulfonic acid with a named pharmaceutical substance (such as compounds of formula (I) or (II)). Use of mesilate rather than mesylate is in compliance with the INNM (International nonproprietary names modified) issued by WHO (e.g., World Health Organization (February 2006). International Nonproprietary Names Modified. INN Working Document 05.167/3. WHO).

As used herein, **"pharmaceutically acceptable solvate"** refers to a molecular complex comprising the 2-aminoarylthiazole derivative of the invention and stoichiometric or sub-stoichiometric amounts of one or more pharmaceutically acceptable solvent molecules such as ethanol. The term **'hydrate'** refers to when said solvent is water.

In one embodiment, the 2-aminoarylthiazole derivative of the invention or a pharmaceutically acceptable salt or solvate thereof is masitinib or a pharmaceutically acceptable salt or solvate thereof.

The chemical name for masitinib is 4-(4-methylpiperazin-1-ylmethyl)-N-[4-methyl-3-(4-pyridin-3ylthiazol-2-ylamino) phenyl]benzamide - CAS number 790299-79-5:

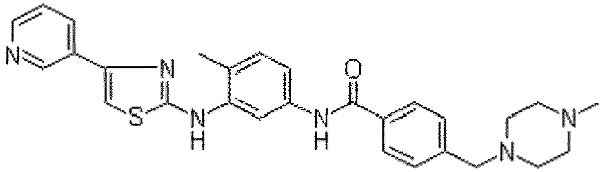

Masitinib was first described in US 7,423,055 and EP 1 525 200 B1.

In one embodiment, the 2-aminoarylthiazole derivative of the invention or a pharmaceutically acceptable salt or solvate thereof is masitinib mesilate. Thus, in one embodiment, the pharmaceutically acceptable salt of masitinib as described hereinabove is masitinib mesilate. As mentioned hereinabove, in other words, the pharmaceutically acceptable salt of masitinib is the methanesulfonic acid salt of masitinib.

A detailed procedure for the synthesis of masitinib mesilate is given in WO 2008/098949.

In one embodiment, "masitinib mesilate" refers to the orally bioavailable mesilate salt of masitinib - CAS 1048007-93-7 (MsOH); C₂₈H₃₀N₆OS.CH₃SO₃H; MW 594.76:

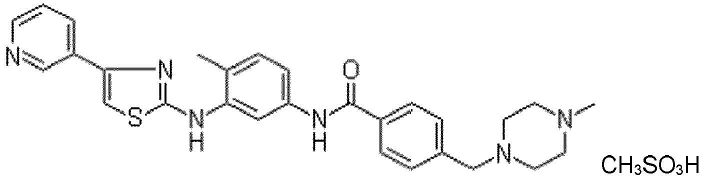

According to one embodiment, the 2-aminoarylthiazole derivative or a pharmaceutically acceptable salt or solvate thereof as described hereinabove, preferably masitinib or a pharmaceutically acceptable salt or solvate thereof, is to be administered at a therapeutically effective dose.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered at a dose ranging from about 1 to about 12 mg/kg/day (mg per kilo body weight per day). In one embodiment, the 2-aminoarylthiazole derivative or a pharmaceutically acceptable salt or solvate thereof as described hereinabove, preferably masitinib or a pharmaceutically acceptable salt or solvate thereof, is to be administered at a dose ranging from about 1.5 to about 7.5 mg/kg/day. In one embodiment, the 2-aminoarylthiazole derivative or a pharmaceutically acceptable salt or solvate thereof as described hereinabove, preferably masitinib or a pharmaceutically acceptable salt or solvate thereof, is to be administered at a dose ranging from about 3 to about 12 mg/kg/day, preferably from about 3 to about 6 mg/kg/day.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered at a dose of about 1, 2, 3, 4, 5, 6, 7, 8, 9, 10, 11 or 12 mg/kg/day. In one embodiment, the 2-aminoarylthiazole derivative or a pharmaceutically acceptable salt or solvate thereof as described hereinabove, preferably masitinib or a pharmaceutically acceptable salt or solvate thereof, is to be administered at a dose of about 1.5, 3, 4.5, 6, 7.5, 9, 10.5 or 12 mg/kg/day.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered at a dose of about 3, 4.5 or 6 mg/kg/day.

In one embodiment, the 2-aminoarylthiazole derivative or a pharmaceutically acceptable salt or solvate thereof as described hereinabove, preferably masitinib or a pharmaceutically acceptable salt or solvate thereof, can be dose escalated by increments of about 1.5 mg/kg/day to reach a maximum of about 7.5 mg/kg/day, more preferably of about 4.5 or about 6 mg/kg/day. Each dose escalation is subjected to toxicity controls with an absence of any toxicity events permitting dose escalation to occur.

In one embodiment, the dose escalation of the 2-aminoarylthiazole derivative or a pharmaceutically acceptable salt or solvate thereof occurs at any time-point after at least 4 weeks after the administration of the initial dose and prior to 26 weeks after the administration of the initial dose; for example at 4 weeks, 8 weeks, 12 weeks, 16 weeks, 20 weeks, or 24 weeks after the administration of the initial dose. Each dose escalation is subjected to toxicity controls, including but not limited to: previous 4-week treatment period at a constant dose of study treatment and no suspected severe adverse event was reported and no suspected adverse event led to treatment interruption and no suspected adverse event is ongoing at the time of the dose increase, regardless of its severity.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered at an initial dose of about 3 mg/kg/day during 6 weeks, then at a dose of about 4.5 mg/kg/day thereafter. In another embodiment, the 2-aminoarylthiazole derivative or a pharmaceutically acceptable salt or solvate thereof as described hereinabove, preferably masitinib or a pharmaceutically acceptable salt or solvate thereof, is to be administered at an initial dose of about 3 mg/kg/day during 12 weeks, then at a dose of about 4.5 mg/kg/day thereafter.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered at an initial dose of about 3 mg/kg/day during at least 4 weeks, then at a dose of about 4.5 mg/kg/day during at least 4 weeks, and at a dose of about 6 mg/kg/day thereafter, with each dose escalation being subjected to toxicity controls. In another embodiment, the 2-aminoarylthiazole derivative or a pharmaceutically acceptable salt or solvate thereof as described hereinabove, preferably masitinib or a pharmaceutically acceptable salt or solvate thereof, is to be administered at an initial dose of about 4.5 mg/kg/day during at least 6 weeks, and at a dose of about 6 mg/kg/day thereafter, with each dose escalation being subjected to toxicity controls.

According to one embodiment, any dose indicated herein refers to the amount of active ingredient as such, not to its pharmaceutically acceptable salt or solvate form. Thus, compositional variations of a pharmaceutically acceptable salt or solvate of the 2-aminoarylthiazole derivative of the invention, in particular masitinib, will not impact the dose to be administered.

According to one embodiment, the 2-aminoarylthiazole derivative or a pharmaceutically acceptable salt or solvate thereof as described hereinabove, preferably masitinib or a pharmaceutically acceptable salt or solvate thereof, may be administered orally, intravenously, parenterally, topically, by inhalation in particular by inhalation spray, rectally, nasally, or buccally.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered orally.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered at least once a day, preferably twice a day.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered for a prolonged period, such as for example, for at least 1, 2, 3, 6, 9, or 12 months.

In one embodiment, the 2-aminoarylthiazole derivative or a pharmaceutically acceptable salt or solvate thereof as described hereinabove, preferably masitinib or a pharmaceutically acceptable salt or solvate thereof, is in a form adapted for oral administration.

Examples of forms adapted for oral administration include, without being limited to, liquid, paste or solid compositions, and more particularly tablets, pills, capsules, liquids, gels, syrups, slurries, and suspensions.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered as tablets, preferably as 100 mg or 200 mg tablets.

According to one embodiment, the 2-aminoarylthiazole derivative or a pharmaceutically acceptable salt or solvate thereof as described hereinabove, preferably masitinib or a pharmaceutically acceptable salt or solvate thereof, is to be administered with at least another pharmaceutically active agent.

According to the present invention, the 2-aminoarylthiazole derivative or a pharmaceutically acceptable salt or solvate thereof as described hereinabove, preferably masitinib or a pharmaceutically acceptable salt or solvate thereof, may be administered simultaneously, separately or sequentially with said at least another pharmaceutically active agent.

In one embodiment, the 2-aminoarylthiazole derivative or a pharmaceutically acceptable salt or solvate thereof as described hereinabove, preferably masitinib or a pharmaceutically acceptable salt or solvate thereof, is to be administered in combination with said at least another pharmaceutically active agent, preferably in a combined preparation, pharmaceutical composition or medicament.

Examples of other pharmaceutically active agents that may be administered to a subject with asthma as described in the present invention include, without being limited to biologics such as anti-IgE agents and anti-cytokine agents, corticosteroids, bronchodilators including long-acting beta agonists (LABAs), rescue medications and any combinations thereof.

In one embodiment, the at least another pharmaceutically active agent is selected from the group comprising or consisting of biologics, corticosteroids, bronchodilators including long-acting beta agonists (LABAs), rescue medications and any combinations thereof.

In one embodiment, the at least another pharmaceutically active agent is a biologic.

As used herein, "a biologic drug", or in short "a biologic", is a compound that is produced from living organisms or contain components of living organisms. Examples of biologics thus include antibodies, recombinant proteins, cells, blood components.

In one embodiment, biologics are selected from anti-IgE agents and anti-cytokine agents such as anti-IL-5 agents. In one embodiment, biologics are antibodies selected from anti-IgE antibodies and anti-cytokine antibodies such as anti-IL-5 antibodies.

In one embodiment, the biologic is an anti-IgE agent. Examples of anti-IgE agents include, without being limited to, the monoclonal antibody omalizumab. In one embodiment, the anti-IgE agent is omalizumab.

In one embodiment, the biologic is an anti-cytokine agent, preferably an anti-cytokine antibody. Examples of anti-cytokine agents include, without being limited to, dupilumab, mepolizumab, reslizumab and benralizumab. In one embodiment, the anti-cytokine agent is selected from the group comprising or consisting of dupilumab, mepolizumab, reslizumab, benralizumab and any mixes thereof.

In one embodiment, the anti-cytokine agent is an anti-IL-5 agent. Thus, in one embodiment, the biologic is an anti-IL5 agent. Anti-IL-5 agents are monoclonal antibodies that target either IL-5 (Interleukin-5) or its receptor (IL-5R).

Examples of anti-IL-5 (interleukin-5) agents include, without being limited to, mepolizumab, reslizumab and benralizumab. In one embodiment, the anti-IL-5 agent is selected from the group comprising or consisting of mepolizumab, reslizumab, benralizumab and any mixes thereof.

In one embodiment, the at least another pharmaceutically active agent is a biologic selected from the group consisting of omalizumab, dupilumab, mepolizumab, reslizumab, benralizumab and any mixes thereof. In one embodiment, the at least another pharmaceutically active agent is a biologic selected from the group consisting of dupilumab, mepolizumab, reslizumab, benralizumab and any mixes thereof. In one embodiment, the at least another pharmaceutically active agent is a biologic selected from the group consisting of mepolizumab, reslizumab, benralizumab and any mixes thereof.

In one embodiment, the at least another pharmaceutically active agent is a corticosteroid. Corticosteroids are anti-inflammatory compounds, administered to reduce airway inflammation.

Examples of corticosteroids include, without being limited to, fluticasone, budesonide, mometasone, beclomethasone, prednisone, prednisolone, betamethasone and dexamethasone.

In one embodiment, the corticosteroid is an inhaled corticosteroid and/or an oral corticoid. In one embodiment, the corticosteroid is selected from the group comprising or consisting of fluticasone, budesonide, mometasone, beclomethasone, prednisone, prednisolone, betamethasone, dexamethasone and any mixes thereof.

In one embodiment, the corticosteroid is an inhaled corticosteroid. Examples of inhaled corticosteroids include, without being limited to, fluticasone, budesonide, mometasone and beclomethasone. In one embodiment, the inhaled corticosteroid is selected from the group comprising or consisting of fluticasone, budesonide, mometasone, beclomethasone and any mixes thereof.

In one embodiment, the corticosteroid is an oral corticosteroid. Examples of oral corticosteroids include, without being limited to, prednisolone, betamethasone and dexamethasone. In one embodiment, the oral corticosteroid is selected from the group comprising or consisting of prednisolone, betamethasone, dexamethasone and any mixes thereof.

In one embodiment, the at least another pharmaceutically active agent is a bronchodilator. Bronchodilators help keep the airways open by relaxing the muscles around the airways.

Examples of bronchodilators include, without being limited to, theophylline and LABAs as described herein. In one embodiment, the bronchodilator is selected from the group comprising or consisting of LABAs as described herein and theophylline.

In one embodiment, the bronchodilator is a LABA. Thus, in one embodiment, the at least another pharmaceutically active agent is a LABA. LABAs are administered to open narrowed airways and prevent asthma exacerbations.

Examples of long-acting beta agonists (which are also known as long-acting β-2 agonists, or long-acting β-2 adrenergic receptor agonists) include, without being limited to, salmeterol, formoterol, arformoterol and vilanterol.

In one embodiment, the LABA is selected from the group comprising or consisting of salmeterol, formoterol, arformoterol, vilanterol and any mixes thereof. In one embodiment, the LABA is selected from the group comprising or consisting of salmeterol, formoterol, arformoterol and any mixes thereof.

In one embodiment, the at least another pharmaceutically active agent is a bronchodilator selected from the group comprising or consisting of theophylline, salmeterol, formoterol, arformoterol, vilanterol and any mixes thereof.

In one embodiment, the at least another pharmaceutically active agent is a combination of a corticosteroid and a LABA. In one embodiment, the LABA is administered with a corticosteroid, in particular with an inhaled corticosteroid.

Examples of combinations of a LABA and an inhaled corticosteroid include, without being limited to, a combination of fluticasone and salmeterol, a combination of budesonide and formoterol, a combination of mometasone and formoterol, a combination of fluticasone and vilanterol.

In one embodiment, the combination of a LABA and an inhaled corticosteroid is selected from the group comprising or consisting of a combination of fluticasone and salmeterol, a combination of budesonide and formoterol, a combination of mometasone and formoterol, a combination of fluticasone and vilanterol and any mixes thereof.

In one embodiment, the at least another pharmaceutically active agent is a leukotriene modifier. Leukotriene modifiers are administered to help relieve asthma symptoms.

Examples of leukotriene modifiers include, without being limited to, montelukast, zafirlukast and zileuton. In one embodiment, the leukotriene modifier is selected from the group comprising or consisting of montelukast, zafirlukast, zileuton and any mixes thereof.

In one embodiment, the at least another pharmaceutically active agent is a rescue medication. In one embodiment, the at least another pharmaceutically active agent is an agent as described above and a rescue medication as described hereinafter.

Rescue medication may be administered as needed, for rapid, short-term symptom relief during an asthma exacerbation. Examples of rescue medication include, without being limited to, bronchodilators such as short-acting beta agonists and ipratropium, corticoids such as oral or intravenous corticosteroids.

Examples of short-acting beta agonists (also known as short-acting beta-2 agonists) include, without being limited to, albuterol (also known as salbutamol) and levalbuterol. Examples of oral or intravenous corticosteroids include, without being limited to, prednisone and methylprednisolone.

In one embodiment, the rescue medication is selected from the group comprising or consisting of albuterol, levalbuterol, ipratropium, prednisone, methylprednisolone and any mixes thereof.

In one embodiment, the at least another pharmaceutically active agent is selected from the group comprising or consisting of omalizumab, dupilumab, mepolizumab, reslizumab, benralizumab, fluticasone, budesonide, mometasone, beclomethasone, prednisone, prednisolone, betamethasone, dexamethasone, theophylline, salmeterol, formoterol, arformoterol, vilanterol, a combination of fluticasone and salmeterol, a combination of budesonide and formoterol, a combination of mometasone and formoterol, a combination of fluticasone and vilanterol, montelukast, zafirlukast, zileuton, albuterol, levalbuterol and any mixes thereof. In one embodiment, the at least another pharmaceutically active agent is selected from the group comprising or consisting of omalizumab, dupilumab, mepolizumab, reslizumab, benralizumab, fluticasone, budesonide, mometasone, prednisone, prednisolone, a combination of fluticasone and salmeterol, a combination of budesonide and formoterol, a combination of mometasone and formoterol, theophylline, albuterol, levalbuterol and any mixes thereof.

In one embodiment, the at least another pharmaceutically active agent is selected from the group comprising or consisting of omalizumab, dupilumab, mepolizumab, reslizumab, benralizumab, prednisone, prednisolone, a combination of fluticasone and salmeterol, a combination of budesonide and formoterol, a combination of mometasone and formoterol and any mixes thereof.

In one embodiment, the 2-aminoarylthiazole derivative as described hereinabove is administered as a first line treatment.

In one embodiment, the 2-aminoarylthiazole derivative as described hereinabove is administered after and/or concomitantly with a treatment with at least another pharmaceutically active agent as described hereinabove. In one embodiment, the 2-aminoarylthiazole derivative as described hereinabove is administered after and/or concomitantly with a treatment with a biologic as described hereinabove. In one embodiment, the 2-aminoarylthiazole derivative as described hereinabove is administered after and/or concomitantly with a treatment with an anti-IL-5 agent as described hereinabove.

In one embodiment, the 2-aminoarylthiazole derivative as described hereinabove is administered as a second line treatment. In one embodiment, the 2-aminoarylthiazole derivative as described hereinabove is administered as a second line treatment, after and/or concomitantly with a first line treatment. In one embodiment, the 2-aminoarylthiazole derivative as described hereinabove is administered as a second line treatment, after and/or concomitantly with a first line treatment comprising the administration of at least another pharmaceutically active agent as described hereinabove. In one embodiment, the 2-aminoarylthiazole derivative as described hereinabove is administered as a second line treatment, after and/or concomitantly with a first line treatment comprising the administration of a biologic as described above. In one embodiment, the 2-aminoarylthiazole derivative as described hereinabove is administered as a second line treatment, after and/or concomitantly with a first line treatment comprising the administration of an anti-IL-5 agent as described hereinabove.

Another object of the present invention is a method for treating asthma in a subject in need thereof, wherein the subject has an elevated level of eosinophils, in particular an elevated level of eosinophils at baseline, as described hereinabove, comprising administering to the subject a 2-aminoarylthiazole derivative or a pharmaceutically acceptable salt or solvate thereof as described hereinabove.

The present invention relates to a method for treating asthma, in particular severe and/or eosinophilic asthma as described hereinabove, in a subject in need thereof, wherein the subject has an eosinophil blood count at baseline equal to or higher than 150 cells/µL, comprising administering to the subject a 2-aminoarylthiazole derivative or a pharmaceutically acceptable salt or solvate thereof as described hereinabove.

The present invention also relates to a method for treating asthma, in particular severe and/or eosinophilic asthma as described hereinabove, in a subject in need thereof, wherein the subject has an eosinophil blood count at baseline ranging from 150 cells/µL to 300 cells/µL, comprising administering to the subject a 2-aminoarylthiazole derivative or a pharmaceutically acceptable salt or solvate thereof as described hereinabove.

The present invention also relates to a method for treating asthma, in particular severe and/or eosinophilic asthma as described hereinabove, in a subject in need thereof, wherein the subject has an eosinophil blood count at baseline equal to or higher than 300 cells/µL, preferably higher than 300 cells/µL, comprising administering to the subject a 2-aminoarylthiazole derivative or a pharmaceutically acceptable salt or solvate thereof as described hereinabove.

In one embodiment, the method of the invention comprises administering to the subject a therapeutically effective dose as described hereinabove of the 2-aminoarylthiazole derivative or a pharmaceutically acceptable salt or solvate thereof of the invention.

In one embodiment, the method of the invention comprises administering to the subject at least another pharmaceutically active agent as described hereinabove.

Another object of the present invention is a pharmaceutical composition comprising a 2-aminoarylthiazole derivative or a pharmaceutically acceptable salt or solvate thereof as described hereinabove and at least one pharmaceutically acceptable excipient for use in the treatment of asthma in a subject in need thereof, wherein the subject has an elevated level of eosinophils, in particular an elevated level of eosinophils at baseline, as described hereinabove.

In one embodiment, the pharmaceutical composition of the invention is for use in combination with at least another pharmaceutically active agent as described hereinabove.

Another object of the present invention is a pharmaceutical composition comprising a 2-aminoarylthiazole derivative or a pharmaceutically acceptable salt or solvate thereof as described hereinabove and at least one pharmaceutically acceptable excipient for the treatment of asthma in a subject in need thereof, wherein the subject has an elevated level of eosinophils, in particular an elevated level of eosinophils at baseline, as described hereinabove.

In one embodiment, the present invention relates to a pharmaceutical composition comprising a 2-aminoarylthiazole derivative or a pharmaceutically acceptable salt or solvate thereof as described hereinabove and at least one pharmaceutically acceptable excipient, in combination with at least another pharmaceutically active agent as described hereinabove, for the treatment of asthma in a subject in need thereof, wherein the subject has an elevated level of eosinophils, in particular an elevated level of eosinophils at baseline, as described hereinabove.

Another object of the present invention is the use of a 2-aminoarylthiazole derivative or a pharmaceutically acceptable salt or solvate thereof as described hereinabove for the manufacture of a medicament for the treatment of asthma as described hereinabove in a subject in need thereof, wherein the subject has an elevated level of eosinophils, in particular an elevated level of eosinophils at baseline, as described hereinabove.

In one embodiment, the present invention relates to the use of a 2-aminoarylthiazole derivative or a pharmaceutically acceptable salt or solvate thereof as described hereinabove for the manufacture of a medicament for the treatment of asthma as described hereinabove in a subject in need thereof in combination with at least another pharmaceutically active agent as described hereinabove, wherein the subject has an elevated level of eosinophils, in particular an elevated level of eosinophils at baseline, as described hereinabove.

In one embodiment, the present invention relates to the use of a 2-aminoarylthiazole derivative or a pharmaceutically acceptable salt or solvate thereof as described hereinabove, in combination with at least another pharmaceutically active agent as described hereinabove, for the manufacture of a medicament for the treatment of asthma in a subject in need thereof, wherein the subject has an elevated level of eosinophils, in particular an elevated level of eosinophils at baseline, as described hereinabove.

### EXAMPLES

The present invention is further illustrated by the following examples.

### Example:

A prospective, multicenter, randomized, double-blind, placebo-controlled, 2-parallel groups, Phase 3 study was carried out to compare the efficacy and the safety of masitinib at 6 mg/kg/day versus placebo in the treatment of subjects with severe persistent asthma treated with oral corticosteroids. Said clinical trial (reference AB07015) is registered under the EudraCT number: 2010-020803-63 and under the ClinicalTrials.gov Identifier: NCT01449162.

### Methods

### Patients

Patients were randomized in 2 groups with a masitinib to placebo randomization ratio of 2:1, respectively:
- Group 1: patients receiving masitinib at 6 mg/kg/day;
- Group 2: patients receiving placebo.

Main inclusion criteria:
- Patient with severe asthma and already treated with oral corticosteroids at a minimal daily dose of 7.5 mg prednisone or equivalent for at least 3 months prior to screening visit;
- Patient with no significant change in the regular asthma medication, no severe asthma exacerbation for at least 4 weeks prior to screening visit;
- Non-smoker patient for at least 1 year and with a prior tobacco consumption < 10 packs/year;
- Patient with history of asthma ≥ 1 year prior to screening visit who also meet the following criteria:
   ∘ baseline FEVi ≥ 35 to < 80% of the predicted normal value, demonstrated at least 6 hours after short-acting Beta-2-agonist or 12 hours after long-acting beta-2-agonist (LABA),
   ∘ at least 2 asthma exacerbations within 1 year prior to screening visit including one severe asthma exacerbation as per protocol definition,
   ∘ uncontrolled asthma as defined as two or more of the following features within the week prior to screening visit (ACQ items):
      ▪ Daytime symptoms more than twice per week,
      ▪ Any limitation of activities symptoms,
      ▪ Any nocturnal symptoms/awakening,
      ▪ Need for reliever/rescue treatment more than twice per week.

Main exclusion criteria:
- Patient with active lung disease other than asthma (*e.g.,* chronic bronchitis);
- Asthmatic patient still exposed to allergens or to triggering factors influencing asthma control; and/or
- Patient with history of acute infectious sinusitis or respiratory tract infection within 4 weeks prior to screening visit.

### Study Design & Treatment Administration

The study consists of the following periods:
- Run-In Phase:
   Patient fulfilling the inclusion/exclusion criteria entered a run-in period consisting of a two-week, single-blind, placebo run-in period. This run-in period served as the reference period for evaluating patient conditions, including symptoms, need of rescue medication and treatment compliance.

In order to be eligible for randomization each patient must demonstrate during the 2-week run in period: good study treatment (*i.e*., placebo) compliance (≥80%); a daily mean symptom score ≥2; and no asthma exacerbation (moderate or severe).
- Initial Treatment Phase (WO-W36):
   During a 36-week treatment period, patients received either masitinib at the fixed dose of 6 mg/kg/day or its matching placebo.
- Oral Corticosteroids Weaning Phase (W36-W44):
   Those patients treated with >15 mg/day of prednisone at week 36 entered an 8-week follow-up oral corticosteroid sparing period. During this period, initial oral corticosteroid dose was decreased of 25% every week until a worsening of asthma occurred. As soon as a patient experienced a worsening of asthma, investigators reintroduced the corticosteroid at the dose of the previous week. No further corticosteroid dose reduction did then take place.
- Extension phase:
   At week 36 for patients ≤15mg/day of prednisone or at week 44 for patient >15 mg/day of prednisone could enter directly a treatment extension phase if judged medically appropriate by the investigator.

Subjects enrolled received a total daily dose of 6 mg/kg of masitinib or matching placebo as indicated in **Table 1,** to be taken during meals.

**Table 1: Dose of study treatment (mg) to be administered according to patient's weight (6 mg/kg/day)**

| **Patient's weight (kg)** | | **Daily dose (6 mg/kg/day)** | **Morning* (mg)** | **Evening** (mg)** |
|---|---|---|---|---|
| > 41.6 | 58.3 | 300 | 100 | 200 |
| > 58.3 | 74.9 | 400 | 200 | 200 |
| > 74.9 | 91.6 | 500 | 200 | 200+100 |
| > 91.6 | | 600 | 200+100 | 200+100 |

Allowed concomitant asthma treatments were:
- Long acting Beta-agonists (wash-out requirement: 12h prior to each study visit);
- Short acting Beta-agonist (salbutamol) as rescue medication, reimbursed by the sponsor (wash-out requirement: 6h prior to each study visit);
- Inhaled corticosteroids at stable dose;
- Oral corticosteroids at stable dose during the first 36 weeks of treatment.

### Efficacy Analysis

According to the statistical analysis plan of study AB07015 (version 1.0 dated 18 Oct 2019), the overall assessable population was defined as: 'All patients in Full Analysis Set (FAS) with severe asthma already treated with oral corticosteroids at a minimal daily dose of greater than 5 mg prednisone or equivalent for at least 3 months prior to screening.'

The full analysis set (FAS) is defined as all randomized patients with exclusion of following patients:
a) Patients from sites with critical Good Clinical Practice (GCP) violations;
b) Patients not treated with investigational product;
c) Patients with randomization error (randomized twice at two different sites);
d) Patients with no reference oral corticosteroid (OCS) dose at screening and baseline.

Subgroup analyses were be performed with cohort categorization of severe asthma patients according eosinophil blood count at baseline.

The primary efficacy endpoint was defined as the severe asthma exacerbation rate adjusted on the available person-time (time to end of treatment) on full exposure (main period + extension / weaning period).

The rate of severe asthma exacerbations was be analyzed by applying a Poisson regression (log-link function and Poisson distribution) model.

A global cut-off for primary analysis was applied at the end of study AB07015, defined as 36 weeks after the last randomization.

Secondary efficacy endpoints included:
- Severe asthma exacerbation rate at week 36;
- Time to first severe asthma exacerbation;
- Percentage of patients experiencing at least one severe asthma exacerbation at week 36 and in overall study;
- Overall (severe and moderate) asthma exacerbation rate at week 36 and in the overall study;
- Time to first asthma exacerbation (severe or moderate);
- Percentage of patients experiencing at least one severe or moderate asthma exacerbation at week 36 and in overall study;
- Asthma Control Questionnaire (ACQ) Score at week 36;
- Asthma Quality of Life Questionnaire (AQLQ) at week 36;
- Forced Expiratory Volume in one second (FEV1) at week 36;
- Forced Vital Capacity (FVC) at week 36;
- Change in the oral corticosteroid doses post baseline at week 36.

### Results

A total of 419 patients were randomized to study AB07015. The Full Analysis Set (FAS) comprised 402 patients; 269 patients in the masitinib treatment-arm and 133 patients in the placebo arm. The assessable population for primary endpoint analysis (*i.e.,* patients with severe asthma already treated with oral corticosteroids at a minimal daily dose of greater than 5 mg prednisone or equivalent for at least 3 months prior to screening) comprised 355 patients; 240 patients in the masitinib treatment-arm and 115 patients in the placebo arm. Of these, a total of 268 patients had an elevated blood eosinophil level equal to or higher than 150 cells/µL; 181 patients in the masitinib treatment-arm and 87 patients in the placebo arm.

Average drug exposure (masitinib or placebo) between treatment-arms was well-matched (*i.e*., about 1:1) for the overall population and across all subgroups. With a masitinib to placebo randomization ratio of 2:1, respectively, the Overall Study Exposure (in years) was also about 2:1; for example, 273 versus 132.5 years, respectively, in the primary analysis population, and 198.6 versus 97.8 years, respectively, in the cohort with elevated blood eosinophil level equal to or higher than 150 cells/µL.

Because eosinophilic inflammation is correlated with asthma severity it was of interest to perform subgroup analyses according to blood eosinophil levels. **Table 2** below presents the results regarding masitinib treatment effect with respect to placebo (rate ratio for asthma exacerbations requiring steroid therapy) in various blood eosinophil level-defined cohorts, and their compliment cohorts (according to the primary efficacy analysis population). The rate of asthma exacerbations is analyzed using Poisson Regression with treatment group (masitinib, placebo) as the primary variable (covariate) of interest.

In the cohort having blood eosinophils equal to or higher than 150 cells/µL (181 versus 87 patients in the masitinib and placebo treatment-arms, respectively) the exacerbation rate for patients receiving masitinib was 0.34 severe exacerbations per year versus 0.48 severe exacerbations per year for placebo-treated patients. The corresponding rate ratio for asthma exacerbations was 0.66, which is equivalent to a 33% reduction (improvement) in severe exacerbation for patients receiving masitinib with respect to placebo-treated patients. In comparison, the compliment cohort, *i.e.,* the subgroup having blood eosinophils less than 150 cells/µL (59 versus 28 patients in the masitinib and placebo treatment-arms, respectively) showed no discernable difference in exacerbation rate with 0.36 versus 0.37 severe exacerbations per year, respectively. The corresponding rate ratio for asthma exacerbations was 0.97, equivalent to a 3% reduction in severe exacerbation for patients receiving masitinib with respect to placebo-treated patients.

Further analyses explored subgroups of the type 2 inflammation severe asthma phenotype. In the cohort having blood eosinophils ranging from 150 to 300 cells/µL (89 versus 35 patients in the masitinib and placebo treatment-arms, respectively), the exacerbation rate for patients receiving masitinib was 0.38 severe exacerbations per year versus 0.53 severe exacerbations per year for placebo-treated patients. The corresponding rate ratio for asthma exacerbations was 0.72, which is equivalent to a 28% reduction (improvement) in severe exacerbation for patients receiving masitinib with respect to placebo-treated patients. Similarly, in the cohort having blood eosinophils equal to or higher than 300 cells/µL (95 versus 52 patients in the masitinib and placebo treatment-arms, respectively), the exacerbation rate for patients receiving masitinib was 0.29 severe exacerbations per year versus 0.50 severe exacerbations per year for placebo-treated patients. The corresponding rate ratio for asthma exacerbations was 0.59, which is equivalent to a 41% reduction (improvement) in severe exacerbation for patients receiving masitinib with respect to placebo-treated patients. In comparison, the aforementioned subgroup having blood eosinophils less than 150 cells/µL showed no discernable difference in exacerbation rate.

From these results it can be concluded that masitinib is beneficial to patients with severe asthma with an elevated level of eosinophils, corresponding to patients with asthma with type 2 inflammation. Of note, masitinib generates a clear treatment effect across said type 2 inflammation severe asthma phenotype, including the difficult to treat subgroup with eosinophils ranging from 150 to 300 cells/µL.

**Table 2: Masitinib treatment effect in patients requiring steroid therapy in terms of yearly exacerbation rate and rate ratio for asthma exacerbations according to blood eosinophil levels**

| Population | Average Exposure (in year) | | Number of Severe Exacerbations | | Yearly Exacerbation Rate | | Rate ratio (Estimate) | Reduction in Severe Exacerbation |
|---|---|---|---|---|---|---|---|---|
| | Masitinib | Placebo | Masitinib | Placebo | Masitinib | Placebo | | |
| Blood eosinophils equal to or higher than 150 cells/µL | 1.10 | 1.12 | 67 (n=39) | 50 (n=24) | 0.34 | 0.48 | 0.66 | 34% |
| (N=268) | | | | | | | | |
| (M: 181, P: 87) | | | | | | | | |
| Blood eosinophils lower than 150 cells/µL | 1.26 | 1.24 | 27 (n=15) | 13 (n=7) | 0.36 | 0.37 | 0.97 | 3% |
| (N=87) | | | | | | | | |
| (M: 59, P: 28) | | | | | | | | |
| Blood eosinophils ranging from 150 to 300 cells/µL | 1.17 | 1.06 | 38 (n=20) | 21 (n=11) | 0.38 | 0.53 | 0.72 | 28% |
| (N=121) | | | | | | | | |
| (M: 86, P: 35) | | | | | | | | |
| Blood eosinophils higher than 300 cells/µL | 1.04 | 1.12 | 29 (n=19) | 29 (n=13) | 0.29 | 0.50 | 0.59 | 41% |
| (N=147) | | | | | | | | |
| (M: 95, P: 52) | | | | | | | | |
| Blood eosinophils equal to or lower than 300 cells/µL | 1.20 | 1.18 | 65 (n=35) | 34 (n=18) | 0.37 | 0.46 | 0.81 | 19% |
| (N=208) | | | | | | | | |
| (M: 145, P: 63) | | | | | | | | |

| | | | | | | | | |
|---|---|---|---|---|---|---|---|---|
| N: total number of subjects; n: number of subjects who experienced severe exacerbation; M: number of subjects who received masitinib; P: number of subjects who received placebo. | | | | | | | | |

## Claims

1. A 2-aminoarylthiazole derivative or a pharmaceutically acceptable salt or solvate thereof for use in the treatment of asthma in a subject in need thereof, wherein the subject has an eosinophil blood count at baseline ranging from 150 cells/µL to 300 cells/µL.

2. The 2-aminoarylthiazole derivative or a pharmaceutically acceptable salt or solvate thereof for use according to claim **1,** wherein the asthma is asthma with type 2 inflammation.

3. The 2-aminoarylthiazole derivative or a pharmaceutically acceptable salt or solvate thereof for use according to claim **1** or claim **2,** wherein the asthma is uncontrolled.

4. The 2-aminoarylthiazole derivative or a pharmaceutically acceptable salt or solvate thereof for use according to any one of claims **1** to **3,** wherein the asthma is uncontrolled with inhaled corticosteroids and/or oral corticosteroids.

5. The 2-aminoarylthiazole derivative or a pharmaceutically acceptable salt or solvate thereof for use according to any one of claims **1** to **4,** wherein the asthma is uncontrolled with biologics.

6. The 2-aminoarylthiazole derivative or a pharmaceutically acceptable salt or solvate thereof for use according to any one of claims **1** to **5,** wherein the 2-aminoarylthiazole derivative has the formula (II): 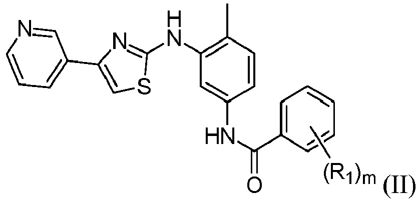 wherein:
- **R₁** is selected independently from hydrogen, halogen, (C₁-C₁₀) alkyl, (C₃-C₁₀) cycloalkyl group, trifluoromethyl, alkoxy, amino, alkylamino, dialkylamino, and a solubilizing group, and
- m is 0-5.

7. The 2-aminoarylthiazole derivative or a pharmaceutically acceptable salt or solvate thereof for use according to any one of claims **1** to **6,** wherein the 2-aminoarylthiazole derivative or a pharmaceutically acceptable salt or solvate thereof is masitinib or a pharmaceutically acceptable salt or solvate thereof.

8. The 2-aminoarylthiazole derivative or a pharmaceutically acceptable salt or solvate thereof for use according to claim **7,** wherein the pharmaceutically acceptable salt or solvate of masitinib is masitinib mesilate.

9. The 2-aminoarylthiazole derivative or a pharmaceutically acceptable salt or solvate thereof for use according to any one of claims **1** to **8,** wherein the 2-aminoarylthiazole derivative or a pharmaceutically acceptable salt or solvate thereof is to be administered orally.

10. The 2-aminoarylthiazole derivative or a pharmaceutically acceptable salt or solvate thereof for use according to any one of claims **1** to **9,** wherein the 2-aminoarylthiazole derivative or a pharmaceutically acceptable salt or solvate thereof is to be administered at a dose ranging from about 1 mg/kg/day to about 12 mg/kg/day, preferably from about 3 mg/kg/day to about 6 mg/kg/day.

11. The 2-aminoarylthiazole derivative or a pharmaceutically acceptable salt or solvate thereof for use according to any one of claims **1** to **10,** wherein the 2-aminoarylthiazole derivative or a pharmaceutically acceptable salt or solvate thereof is to be administered at a dose of about 3 mg/kg/day, 4.5 mg/kg/day or 6 mg/kg/day.

12. The 2-aminoarylthiazole derivative or a pharmaceutically acceptable salt or solvate thereof for use according to any one of claims **1** to **11,** wherein the 2-aminoarylthiazole derivative or a pharmaceutically acceptable salt or solvate thereof is to be administered with at least another pharmaceutically active agent.

13. The 2-aminoarylthiazole derivative or a pharmaceutically acceptable salt or solvate thereof for use according to claim **12,** wherein the at least another pharmaceutically active agent is selected from the group consisting of biologics, corticosteroids, bronchodilators, leukotriene modifiers, rescue medications and any combinations thereof.

14. The 2-aminoarylthiazole derivative or a pharmaceutically acceptable salt or solvate thereof for use according to claim **12** or claim **13,** wherein the at least another pharmaceutically active agent is a biologic selected from the group consisting of anti-IgE agents and anti-cytokine agents.

15. The 2-aminoarylthiazole derivative or a pharmaceutically acceptable salt or solvate thereof for use according to claim **14,** wherein the biologic is an anti-IL-5 agent.
